# EUROPÄISCHE PATENTSCHRIFT

(11) **EP 2 572 647 B1**
(45) Veröffentlichungstag und Bekanntmachung des Hinweises auf die Patenterteilung: **09.01.2019**
(21) Anmeldenummer: 12185708.0
(22) Anmeldetag: 24.09.2012
(51) Int. Cl.: A61B 17/02, A61B 17/00

(54) **Chirurgisches Retraktionssystem**
Surgical retraction system
Système de rétraction chirurgical

(30) Priorität: 26.09.2011 DE 102011053938; 17.11.2011 DE 202011051999 U
(43) Veröffentlichungstag der Anmeldung: 27.03.2013
(73) Patentinhaber: AESCULAP AG, 78532 Tuttlingen (DE)
(72) Erfinder: Weißhaupt, Dieter, 78194 Immendingen (DE); Morales, Pedro, 78532 Tuttlingen (DE); Rothweiler, Christoph, 78166 Donaueschingen (DE); Vogtherr, Robert, 78532 Tuttlingen (DE)
(74) Vertreter: Hoeger, Stellrecht & Partner Patentanwälte mbB

(56) Entgegenhaltungen:
- WO-A1-98/12960
- WO-A1-2004/105619
- WO-A1-2008/131355
- DE-U1- 20 003 335
- US-A- 5 365 921
- US-A- 5 908 382
- US-A- 6 139 493
- Spectravision Meditech: "Spectravision Meditech online catalogue for McCulloch Spinal Retractor", , 11 April 2011 (2011-04-11), XP055445641, Internet Retrieved from the Internet: URL:http://www.spectravision.co.in/pdf/spi ne_mcculloch_spinal_retractor.pdf [retrieved on 2018-01-29]

## Beschreibung

Die Erfindung betrifft ein chirurgisches Retraktionssystem, insbesondere zum Spreizen eines durchtrennten Sternums, mit einer Haltevorrichtung, zwei Spreizarmen, welch letztere jeweils mit einem ersten Ende an der Haltevorrichtung in einem in einer Spreizebene veränderlichen Abstand voneinander gehalten sind, sowie mindestens zwei Rückhalteelementen, die an den zu retrahierenden Knochenmaterialien angreifen und diese halten. An jedem der Spreizarme ist jeweils mindestens ein Rückhalteelement angeordnet. Häufig sind die Spreizarme gerade ausgebildet.

Vorrichtungen der eingangs genannten Art werden häufig in der Herz-Thorax-Chirurgie verwendet, wenn Durchblutungsstörungen von Herzkranzgefäßen behandelt werden sollen. Eine der gängigen Methoden zur Behandlung von Durchblutungsstörungen der Herzkranzgefäße ist die so genannte Bypass-Operation. Diese wird nötig, wenn sich die Verengungen in den Herzkranzgefäßen nicht durch Medikamente oder andere Therapiemethoden, wie zum Beispiel eine Ballon-Dilatation, behandeln lassen.

Bei der klassischen Bypass-Operation wird zuerst das Sternum eröffnet, d.h. in Längsrichtung ungefähr mittig durchtrennt. Die Sternumhälften mit den daran anschließenden Rippen werden auseinandergedrängt, um einen operativen Zugang zu schaffen. Nun gibt es mehrere Möglichkeiten zur operativen Überbrückung der Verengung (Stenose) der Herzkranzgefäße. Eine der am weitesten verbreiteten Operationsmethoden ist die Schaffung eines so genannten Arteria-Mammaria-Bypass (Arterien-Bypass).

Die an der Innenseite des Brustkorbes verlaufenden, arteriellen Blutgefäße, die Arteria Mammaria Interna (links = LIMA und rechts = RIMA), sind ähnlich groß wie die Herzkranzgefäße. Der Operateur legt normalerweise die linksseitige Arteria Mammaria Interna ein Stück weit frei und schließt deren Ende jenseits der Verengung an die Koronararterie an. Falls es erforderlich ist, wird auch die rechte Arteria Mammaria Interna verwendet. Bei dieser Methode ist die Gefahr eines erneuten Verschlusses des Umgehungskreislaufs kleiner als bei einem venösen Bypass.

Die Kräfte, die beim Spreizen des Brustkorbes bzw. des Sternums mit den daran anschließenden Rippen aufgewendet werden müssen, sind oft sehr groß, so dass die auftretenden Verformungen der Rippen Absplitterungen oder Rippenbrüche nach sich ziehen und postoperativ erhöhte Schmerzen bei den Patienten verursachen können.

Aus der DE 200 03 335 U1 ist ein Retraktor mit einer Schiene bekannt, von der ein erster Haltearm abgewinkelt absteht, sowie mit einem längs der Schiene über einen Antriebsmechanismus verfahrbaren beweglichen zweiten Haltearm, der sich etwa parallel zum ersten Haltearm erstreckt und auf diesen zu bzw. von diesem weg bewegbar ist. An den Haltearmen sind lösbar anbringbare Fuktionselementen vorgesehen, wobei jedes Funktionselement auf einem Haltearm stufenlos längsverschieblich ist, dort unverlierbar aufgenommen ist und durch den Spreizgegendruck in einer Verschiebeposition fixierbar ist.

Die WO 2008/131355 A1 beschreibt einstellbare Rückhalteflächen eines Retraktors, bei denen ein vertikaler Abschnitt der Rückhaltefläche mit einem einstellbaren Befestigungsteil integral verbunden ist, mit dem die Rückhaltefläche an einem Arm eines Retraktors festlegbar ist. Das einstellbare Befestigungsteil erlaubt die Anpassung der Position des vertikalen Abschnitts im Körper eines Patienten.

Das US-Patent US 5,908,382 A offenbart einen Retraktor mit einem Retraktor-Rahmen, welcher einen feststehenden Arm und einen beweglichen Arm aufweist. Ein Hebelmechanismus erlaubt die Anpassung der Position des beweglichen Arms gegenüber dem feststehenden Arm. An dem beweglichen Arm sind anpassbare Rückhalteelemente vorgesehen, die es erlauben den Winkel des Retraktorquerarms nach dem Einsetzen des Rückhalteelements in einer Inzision anzupassen.

Aus dem US-Patent US 6,139,493 A sind Retraktoren mit Rückhalteelementen bekannt, deren Länge angepasst werden kann.

Ferner offenbart die WO 2004/105619 A1 einen Retraktor mit einer Schiene, von der ein erster Haltearm abgewinkelt absteht. Der Retraktor weist einen zweiten Haltearm auf, der sich parallel zum ersten Haltearm mit veränderbarem Abstand erstreckt. An den Haltearmen sind verschwenkbare Funktionselemente gehalten, deren Schwenkstellung einstellbar und fixierbar ist.

Dokumente US5365921 A und WO9812960 A1 offenbaren weitere Retraktoren aus dem Stand der Technik.

Die Erfindung wird durch den anhängenden unabhängigen Anspruch 1 beschränkt. Weitere bevorzugte Ausführungsformen können den abhängigen Ansprüchen entnommen werden.

Aufgabe der vorliegenden Erfindung ist es, ein Retraktionssystem der eingangs beschriebenen Art so weiterzubilden, dass es einfach und für den Patienten möglichst wenig belastend einsetzbar ist und insbesondere auch bei der Arteria-Mammaria-Präparation ein zeitsparenderes und sichereres Arbeiten möglich ist.

Diese Aufgabe wird Erfindung gemäß dadurch gelöst, dass das System Rückhalteelemente aufweist, die mehrteilig ausgebildet sind, wobei ein erstes Teil der Rückhalteelemente einen Lagerkörper bildet, der am Spreizarm gehalten ist, und wobei an dem ersten Teil ein zweites Teil des Rückhalteelements in Form einer Rückhaltefläche um eine im Wesentlichen senkrecht zur Spreizebene des Retraktionssystems ausgerichtete Achse drehbar gehalten ist.

Aufgrund der drehbaren Lagerung der Rückhalteflächen der Rückhalteelemente an den Spreizarmen des Retraktionssystems können sich die Rückhalteflächen an die jeweils gegebenen Anlageflächen seitens des kontaktierten Knochenmaterials, insbesondere des durchtrennten Sternums oder, im Falle der Retraktion von Rippen, der jeweils betroffenen Rippen, großflächig anlegen, so dass eine schonendere Retraktion der Knochenmaterialien möglich ist. Diese Anpassung geschieht, ohne dass der Chirurg eingreifen oder nachjustieren muss.

Eine noch bessere Anpassung der Lage der Rückhalteflächen wird dadurch erzielt, dass ein Lagerkörper von mindestens einem der Rückhalteelemente die Rückhaltefläche um eine zweite Achse schwenkbar hält, wobei die zweite Achse im Wesentlichen senkrecht zur ersten Achse und zur Rückhaltefläche ausgerichtet ist. Auch hier ergibt sich die Anpassung, ohne dass der Chirurg eingreifen oder nachjustieren muss.

Weiterhin ist erfindungsgemäß die Rückhaltefläche von mindestens einem der Rückhalteelemente in zwei Bereiche geteilt ausgebildet, wobei der erste Bereich der Rückhaltefläche an dem Lagerkörper gelenkig um die erste Achse drehbar und gegebenenfalls um die zweite Achse schwenkbar gehalten ist und wobei der zweite Bereich an dem ersten Bereich um eine dritte Achse schwenkbar gehalten ist, wobei die dritte Achse im Wesentlichen parallel zur Rückhaltefläche und im Wesentlichen senkrecht zur ersten Achse ausgerichtet ist.

Bei einer ersten Variante dieser Ausführungsform ist der erste Bereich mit einer Ausnehmung versehen, welche sich vom Bereich der dritten Achse in Richtung zum Lagerkörper erstreckt, so dass sich insbesondere eine gabelförmig ausgebildete Ausnehmung ergibt. Der zweite Bereich der Rückhaltefläche weist einen zur Ausnehmung des ersten Bereichs im Wesentlichen komplementären Vorsprung auf. Für eine bessere Handhabung kann vorgesehen sein, dass am ersten Bereich ein Anschlag ausgebildet ist, an dem der zweite Bereich zur Anlage bringbar ist. Der Schwenkwinkel des zweiten Bereichs gegenüber dem ersten Bereich ist damit beschränkt. Weiter bevorzugt weist der erste Bereich einen weiteren Anschlag auf, an dem der zweite Bereich zur Anlage bringbar ist. Damit ist der Schwenkwinkel des zweiten Bereichs in beiden möglichen Schwenkrichtungen begrenzbar.

Beispielsweise kann ein erster Anschlag im Bereich der Ausnehmung des ersten Bereichs vorgesehen sein, an dem dann der Vorsprung des zweiten Bereichs in einer ersten Schwenkposition zur Anlage bringbar ist. Der weitere Anschlag kann beispielsweise benachbart zum Schwenklager des ersten Bereichs ausgebildet sein, an dem der zweite Bereich der Rückhaltefläche in einer zweiten Schwenkposition zur Anlage bringbar ist.

Bei einer zweiten Variante dieser Ausführungsform weist der erste Bereich der Rückhaltefläche einen Vorsprung auf, welcher an seinem freien Ende ein Lager für die dritte Achse umfasst, wobei der zweite Bereich der Rückhaltefläche eine zu dem Vorsprung des ersten Bereichs im Wesentlichen komplementäre Ausnehmung aufweist, so dass sich vorzugsweise eine gabelförmige Konfiguration ergibt. Vorzugsweise ist auch bei dieser Variante am ersten Bereich der Rückhalte-fläche ein Anschlag, optional auch ein weiterer Anschlag, ausgebildet, an dem der zweite Bereich jeweils zur Anlage bringbar ist, so dass wie bei der ersten Variante der Schwenkwinkel des zweiten Bereichs gegenüber dem ersten Bereich gegebenenfalls in beiden möglichen Schwenkrichtungen begrenzbar ist.

Bei weiteren bevorzugten Ausführungsformen der vorliegenden Erfindung ist die Rückhaltefläche mindestens eines der Rückhalteelemente in zwei Bereiche geteilt ausgebildet, wobei der erste Bereich am Lagerkörper gelenkig gehalten ist und wobei der zweite Bereich gegenüber dem ersten Bereich in einer Ebene parallel zur ersten Achse bewegbar gelagert ist. Vorzugsweise ist der zweite Bereich in unterschiedlichen Abständen zum Lagerteil des Rückhalteelements entlang der ersten Achse positionierbar.

Damit lassen sich die Rückhalteflächen an die jeweiligen Gegebenheiten, d.h. die an dem Rückhalteelement anliegenden Knochenmaterialien und deren Ausdehnung senkrecht zur Spreizebene, anpassen.

Besonders bevorzugt ist, dass einer der Bereiche vorzugsweise am anderen Bereich um eine Achse senkrecht zur Drehachse des Rückhalteelements schwenkbar gelagert ist. Mit dieser Maßnahme wird zudem erreicht, dass sich die Rückhaltefläche auch dann optimal an das in Kontakt stehende Knochenmaterial anpasst, wenn das Retraktionssystem mit seiner Spreizebene insgesamt bewegt werden muss.

Bevorzugt ist die Rückhaltefläche eines Rückhalteelements zum ersten Teil oder Lagerkörper des Rückhalteelements hin trapezförmig verjüngt ausgebildet. Dies hat den Vorteil, dass zum einen der Platzbedarf des Rückhalteelements in der Ebene der Spreizarme relativ gering ist und zum anderen in dem Bereich der Rückhalteflächen ein großflächiger Kontakt mit dem zu retrahierenden Knochenmaterial möglich ist.

Bei einer weiteren bevorzugten Ausführungsform der Rückhalteelemente weist die Rückhaltefläche an ihrem freien Ende eine geringere Ausdehnung senkrecht zur Drehachse auf als in einem davon entfernten Bereich.

Insbesondere kann das freie Ende der Rückhaltefläche zu einem stegartigen Vorsprung verjüngt ausgebildet sein.

Weiter bevorzugt ist das freie Ende der Rückhaltefläche als abgewinkelter Abschnitt ausgebildet, der dann unter das zu retrahierende Knochenmaterial greift. Besonders bevorzugt weist die Rückhaltefläche einen U-förmigen Querschnitt auf.

Bei bevorzugten Systemen sind an jedem Spreizarm mindestens zwei Rückhalteelemente angeordnet. Dabei ist es bevorzugt, dass die beiden Rückhalteelemente, in Längsrichtung der Spreizarme gesehen, versetzt gegeneinander angeordnet sind. Dies lässt eine größtmögliche Annäherung der Spreizarme zu, so dass ein Einführen des Systems in ein frisch durchtrenntes Sternum mit geringem Platzbedarf möglich ist.

Weiter bevorzugt ist es, dass das oder die Rückhalteelemente in ihrem Abstand zur Haltevorrichtung entlang des Spreizarms verstellbar sind.

Um einen optimalen Zugang zu dem Operationsfeld, insbesondere für die Arteria-Mammaria-Präparation, zu bekommen, werden oft unterschiedliche Spreizsysteme eingesetzt, die ein- und ausgebaut werden müssen, was den Operationsfluss unterbricht. Darüber hinaus besteht durch das Einbringen und das Herausnehmen der unterschiedlichen Spreizsysteme eine Verletzungsgefahr für den Patienten.

Hier kann ein Spreizsystem, das ein Spreizen des Brustkorbes und ein Anheben einer Brustkorbseite zur Arteria-Mammaria-Präparation erlaubt, Abhilfe schaffen. Ein solches Spreizsystem ist zum Beispiel aus dem U.S.-Patent 6,416,468 B2 bekannt.

Bei diesem aus dem U.S.-Patent 6,416,468 B2 bekannten Spreizsystem erfolgt gleichzeitig ein Spreizen der von dem Spreizsystem erfassten Knochenmaterialien und ein Anheben einer der Seiten der eröffneten Operationswunde. Die Vorrichtung lässt sich auch umrüsten, so dass nur eine Spreizung möglich ist.

Nachteilig bei dieser Spreizvorrichtung gemäß dem U.S.-Patent 6,416,468 B2 ist, dass im Falle, dass eine Anhebung notwendig wird, diese nicht unabhängig von der Spreizung der Operationswunde möglich ist. Diese zwangsweise gekoppelten Vorgänge erfordern relativ hohe Kräfte, so dass der Vorgang oft nicht mit der gewünschten Feinfühligkeit ausgeführt werden kann. Eine zu große und/oder zu schnelle Dehnung oder gar ein Bruch der Knochensubstanz kann die Folge sein.

Die Erfindung schlägt bei dem eingangs beschriebenen chirurgischen Retraktionssystem in diesem Zusammenhang vor, dass das System ferner eine Hebevorrichtung aufweist, wobei die Hebevorrichtung ein sich im Wesentlichen parallel zur Spreizebene des Systems erstreckendes Halteelement, welches mit einem ersten Ende an einem der Spreizarme - vorzugsweise lösbar - gehalten ist, und eine sich vom Halteelement aus erstreckende Stützvorrichtung mit einer am freiem Ende der Stützvorrichtung gehaltenen Stützplatte zur Anlage am Brustkorb des Patienten umfasst, wobei die Stützvorrichtung die Stützplatte in einem variablen, vorgebbaren Abstand zur Spreizebene hält. Die Stützvorrichtung erstreckt sich vorzugsweise in einer Ebene, die senkrecht zur Spreizebene ausgerichtet ist.

Bei einem solchen Retraktionssystem der vorliegenden Erfindung eignen sich insbesondere die zuvor beschriebenen Rückhalteelemente.

Bevorzugt weist das chirurgische Retraktionssystem eine Stützvorrichtung auf, die eine Schwenkvorrichtung mit einem Schwenkarm und einem Abstandhalter umfasst. Der Schwenkarm ist mit einem ersten Ende gelenkig an dem Halteelement der Hebevorrichtung, benachbart zum Spreizarm, montiert und trägt an seinem entgegengesetzten, zweiten Ende das Stützelement. Der Abstandhalter ist mit einem ersten Ende benachbart zum zweiten Ende des Halteelements an diesem gehalten und stützt sich mit seinem zweiten Ende an dem Schwenkarm ab und hält so das das Stützelement tragende zweite Ende des Schwenkarms in einem variabel vorgebbaren Abstand zum zweiten Ende des Halteelements.

Bevorzugt ist der Abstandhalter längenveränderlich ausgebildet, so dass dieser den Abstand zwischen dem zweiten Ende des Halteelements und dem zweiten Ende des Schwenkarms in unterschiedlichen Positionen fixieren kann.

Eine weitere bevorzugte Ausführungsform des erfindungsgemäßen Retraktionssystems beinhaltet einen Abstandhalter, der mit einem ersten Ende benachbart zu dem zweiten Ende des Halteelements an diesem gelenkig gehalten ist, wobei an dem Schwenkarm mehrere Raststellungen ausgebildet sind, in denen das zweite Ende des Abstandhalters lösbar positionierbar ist.

Bei einer weiteren Ausführungsform des erfindungsgemäßen chirurgischen Retraktionssystems ist vorgesehen, dass der Abstandhalter mit einem ersten Ende benachbart zum zweiten Ende des Schwenkarms gelenkig gehalten ist, wobei an dem Halteelemente mehrere Raststellungen ausgebildet sind, in denen das zweite Ende des Abstandhalters lösbar positionierbar ist.

Die Raststellungen der vorgenannten beiden Alternativen von bevorzugten Ausführungsformen können als Zahnstangensegmente ausgebildet werden.

Weiter bevorzugt ist bei dem erfindungsgemäßen chirurgischen Retraktionssystem das Halteelement an seinem zweiten Ende mit einem Griffelement versehen.

Ein weiterhin bevorzugtes chirurgisches Retraktionssystem der vorliegenden Erfindung zeichnet sich dadurch aus, dass das Halteelement benachbart zu seinem zweiten Ende eine Langlochöffnung aufweist, in der der Abstandhalter mit seinem ersten Ende schwenkbar gehalten ist. Durch die Langlochöffnung wird eine gewisse Führung des Abstandhalters bei seiner Verschwenkbewegung erzielt.

Bei einer weiter bevorzugten Ausführungsform des erfindungsgemäßen chirurgischen Retraktionssystems weist der Abstandhalter an seinem ersten Ende ein Griffteil auf, welches durch die Langlochöffnung des Halteelements hindurch reicht. Damit lässt sich mit einer Hand das Retraktionssystem anheben und der Abstandhalter beim Erreichen der gewünschten angehobenen Position in seine Raststellung am Schwenkarm platzieren.

Diese und weitere Vorteile der Erfindung werden im Folgenden anhand der Zeichnung noch näher erläutert. Es zeigen im Einzelnen:
- Figur 1:: ein chirurgisches Retraktionssystem (Referenz);
- Figuren 2A bis 2D:: verschiedene Ausführungsformen von Rückhalteelementen für ein chirurgisches Retraktionssystem gemäß Figur 1; Figuren 2A und 2B dienen als Referenz; Figuren 2C und 2D zeigen Rückhalteelemente für ein Retraktionssystem;
- Figur 3:: ein chirurgisches Retraktionssystem gemäß einer Ausführungsform;
- Figuren 4A und 4B:: das Retraktionssystem der Figur 3 in Drauf- und Seitenansicht;
- Figur 5:: einen Hebelmechanismus zum Verschwenken eines erfindungsgemäßen chirurgischen Retraktionssystems;
- Figur 6:: ein chirurgisches Retraktionssystem (Referenz);
- Figur 7:: das chirurgische Retraktionssystem gemäß Figur 6 in einer alternativen Konfiguration;
- Figuren 8A bis 8D:: eine Ausführungsform eines Rückhalteelements für ein erfindungsgemäßes chirurgisches Retraktionssystem; und
- Figuren 9A bis 9C:: eine weitere Ausführungsform eines Rückhalteelements für ein erfindungsgemäßes chirurgisches Retraktionssystem.

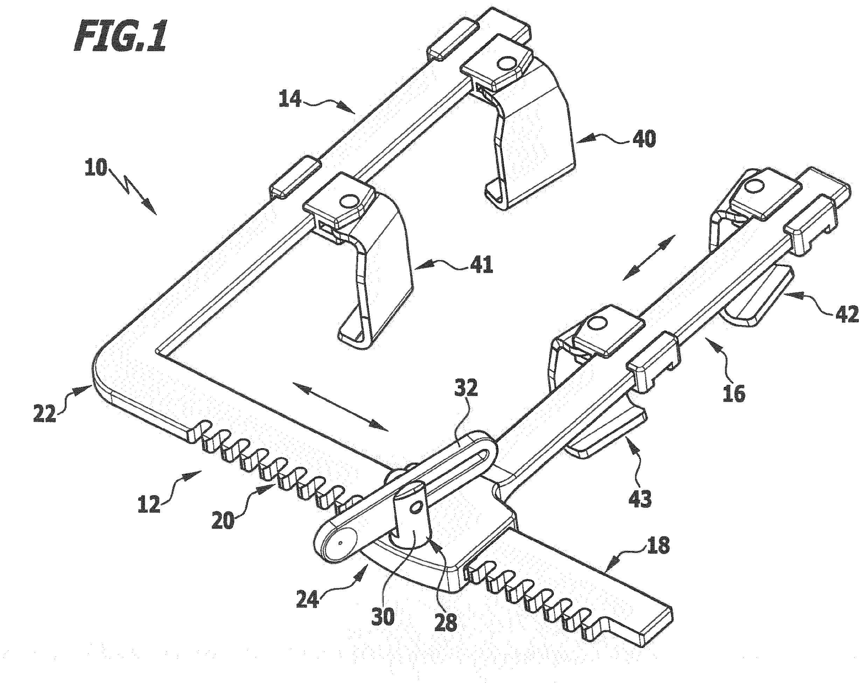

Figur 1 zeigt eine erste Ausführungsform eines insgesamt mit dem Bezugszeichen 10 versehenen chirurgischen Retraktionssystems mit einer Haltevorrichtung 12, einem ersten Spreizarm 14 und einem zweiten Spreizarm 16. Die beiden Spreizarme 14 und 16 sind im Wesentlichen gerade ausgebildet und liegen in einer Ebene, die parallel zu der Spreizebene liegt.

Die Haltevorrichtung 12 weist eine typischerweise gerade Schiene 18 mit einem im Wesentlichen rechteckigen Querschnitt auf, bei welcher in einem Abschnitt an einer Schmalseite eine Zahnreihe 20 ausgebildet ist.

An einem Ende 22 der Haltevorrichtung 12 ist der erste Spreizarm 14 im rechten Winkel zur Längsrichtung der Schiene 18 einstückig angeformt. Der erste Spreizarm 14 weist ebenfalls einen im Wesentlichen rechteckigen Querschnitt auf.

Der zweite Spreizarm 16 weist ebenfalls einen rechteckigen Querschnitt auf und ist mit einem Ende an der Haltevorrichtung 12 parallel zum ersten Spreizarm 14 und diesem gegenüber beweglich mittels eines Lagerelements 24 gehalten, so dass der Abstand der beiden Spreizarme 14 und 16 voneinander in der Spreizebene bei Bedarf verändert werden kann.

Das Lagerelement 24 des Spreizarms 16 umfasst einen Antriebsmechanismus 28 mit einem im Einzelnen nicht dargestellten Drehkörper, beispielsweise ein Zahnrad oder ein Zapfenrad, welcher mit der Zahnreihe 20 in Eingriff steht. Der Drehkörper ist über eine in dem Lagerelement 24 senkrecht zur Spreizebene drehbar gehaltenen Welle 30 drehfest mit einem Griffteil 32 verbunden. Durch Drehen des Griffteils 32 lässt sich der Spreizarm 16 entlang der Schiene 18 bewegen und so der Abstand zwischen den beiden Spreizarmen 14 und 16 in der Spreizebene vergrößern oder verkleinern.

Der Antriebsmechanismus 28 des Spreizarms 16 dient zum einen zur Bewegung des Spreizarms 16, insbesondere unter Last, beispielsweise beim Retrahieren der Hälften eines Sternums mit den daran anschließenden Rippen eines Patienten, entlang der Zahnreihe 20 der Haltevorrichtung 12 und zum anderen auch der Sicherung des Spreizarms 16 in der dann erreichten Abstandsposition zu dem Spreizarm 14. Der Antriebsmechanismus 28 ist deshalb vorzugsweise selbsthemmend ausgebildet.

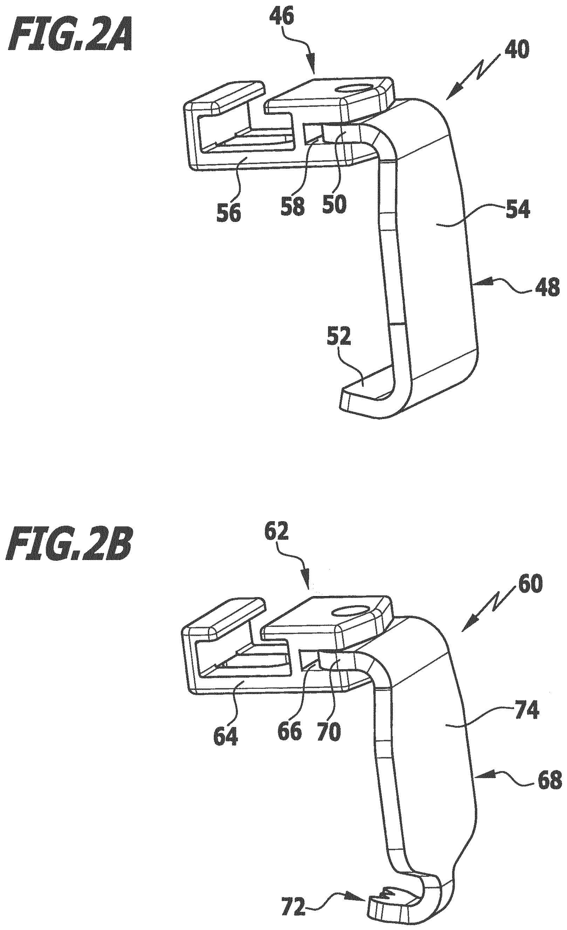

An den beiden Spreizarmen 14, 16 sind jeweils zwei Rückhalteelemente 40, 41 bzw. 42, 43 angeordnet. Die Ausgestaltung der Rückhalteelemente 40, 41, 42, 43 wird anhand der Figur 2A im Folgenden im Einzelnen am Beispiel des Rückhalteelements 40 beschrieben.

Das Rückhalteelement 40 ist zweiteilig ausgebildet, wobei ein erstes Teil als Lagerkörper oder Halteteil 46 der Montage des Rückhalteelements an dem jeweils zugeordneten Spreizarm 14 bzw. 16 dient. Ein zweiter Teil 48 bildet eine Rückhaltefläche, im Folgenden auch Valve genannt, die eine Anlagefläche für zu retrahierendes Knochenmaterial bildet.

Die Valve 48 weist einen U-förmigen Querschnitt auf mit einem ersten Schenkel 50, einem zweiten, gegenüberliegenden Schenkel 52 und einem dazwischen angeordneten, im Wesentlichen planaren, mittleren Abschnitt 54.

Das erste Teil 46 des Rückhalteelements 40, das als Halteteil fungiert, weist in einem ersten Bereich ein im Wesentlichen rechteckiges, nach oben offenes Hohlprofil 56 auf, das im Wesentlichen dem Querschnitt des Spreizarms 14 bzw. 16 entspricht und diesen gleitend aufnehmen kann. An einer der Schmalseiten weist das Halteteil 46 einen sich lateral öffnenden C-förmigen Querschnittbereich 58 auf.

Das Halteteil 46 nimmt mit seinem C-förmigen Querschnittsbereich 58 den ersten Schenkel 50 der Valve 48 auf, wobei der Schenkel 50 in dem C-förmigen, lateral offenen Querschnittsbereich 58 um eine Achse, die senkrecht zur Spreiz-ebene des Systems 10 ausgerichtet ist, drehbar gehalten ist.

Der planare mittlere Abschnitt 54 liegt beim Retrahieren typischerweise an dem Knochenmaterial an. Der zweite Schenkel 52 umgreift dabei das Knochenmaterial und hält dieses so sicher innerhalb des U-förmigen Querschnitts des Rückhalteelements 40.

Figur 2B zeigt ein alternatives Rückhalteelement 60 mit einem ersten als Halteteil 62 ausgebildeten Teil und einem zweiten als Rückhaltefläche ausgebildeten Teil 68. Das Halteteil 62 weist einen hohlprofilartigen Abschnitt 64 auf, an den sich seitlich ein C-förmig profiliertes Teil 66 anschließt, das sich lateral öffnet.

Das zweite Teil 68 weist einen U-förmigen Querschnitt auf, wobei ein erster Schenkel 70 sich von einem im Wesentlichen planaren mittleren Abschnitt 74 weg erstreckt und in das C-förmige Profil 66 des ersten Halteteils 62 eingreift. Der Schenkel 70 ist in dem C-förmigen Profil 66 um eine senkrecht zur Spreizebene ausgerichtete Achse drehbar gelagert. Der zweite Schenkel des U-förmigen Profils des Teils 68 ist als stegartiger abgewinkelter Vorsprung 72 ausgebildet.

Aufgrund der geringen lateralen Ausdehnung des Schenkels 72 ist im Bereich der Arteria Mammaria ausreichend Sicht für den Chirurgen gegeben, wenn diese präpariert werden muss.

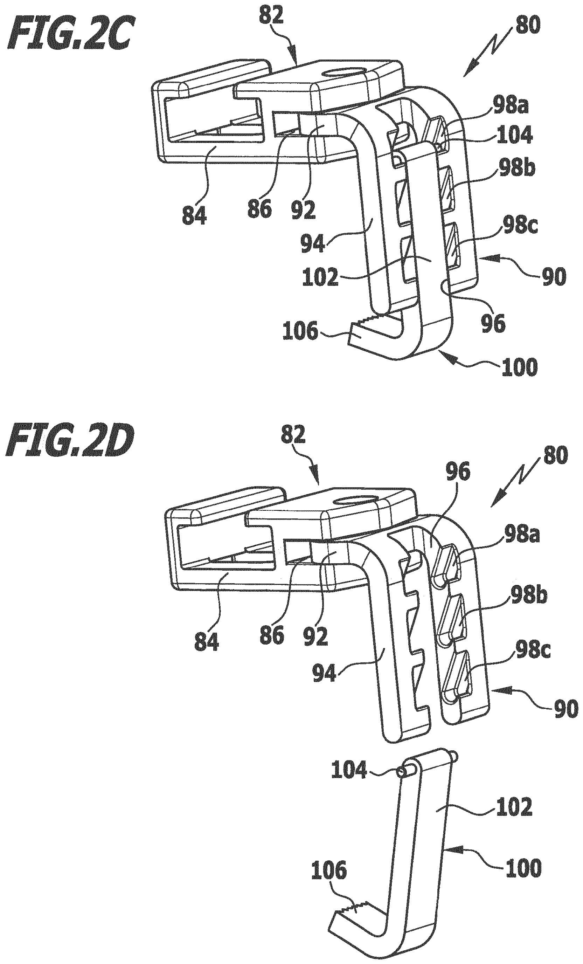

Eine weitere Alternative eines Rückhalteelements 80 ist in den Figuren 2C und 2D dargestellt. Das Rückhalteelement 80 ist dreiteilig aufgebaut, wobei ein erstes Teil als Halteteil 82 ausgebildet und ähnlich aufgebaut ist wie die Halteteile 46 und 62 der Rückhalteelemente 40 und 60, die in den Figuren 2A und 2B gezeigt sind.

Das Halteteil 82 umfasst dabei ein nach oben offenes Hohlprofil 84, dessen lichter Querschnitt im Wesentlichen dem Querschnitt der Spreizarme 14 bzw.

16 entspricht und diese gleitend aufnehmen kann. Seitlich ist an dem Hohlprofil 84 ein lateral offenes C-förmiges Profil 86 angeformt.

Ein zweites Teil des Rückhalteelements 80 ist mit dem Bezugszeichen 90 versehen und L-förmig ausgebildet, wobei ein kurzer Schenkel 92 in das C-förmige Profil 86 eingreift und dort um eine Achse senkrecht zur Spreizebene drehbar gelagert ist. Der lange Schenkel 94 erstreckt sich senkrecht zur Spreizebene und dient als Anlage für das Knochenmaterial. Der lange Schenkel 94 ist mit einer zu seinem freien Ende hin offenen Nut 96 versehen. Die Nut 96 ist in regelmäßigen Abständen mit seitlichen Ausnehmungen, vorzugsweise in Schlitzform, versehen, wobei die Längsrichtung der Schlitze einen spitzen Winkel zu der Längsrichtung des Schenkels 94 bildet. Die Schlitze bilden Lagerstellen 98a, 98b und 98c.

Das Rückhalteelement 80 umfasst ein drittes Teil 100, welches ebenfalls L-förmig ausgebildet ist und am Ende seines langen Schenkels 102 einen seitlich jeweils überstehenden Stift als Lagerwelle 104 aufnimmt. Die Welle 104 kann wahlweise in die Lagerstellen 98a, 98b und 98c eingesetzt werden und hält dann das dritte Teil 100 schwenkbar und von der Nut 96 geführt. Der kurze Schenkel 106 des dritten Teils 100 umgreift wieder das Knochenmaterial.

Das Rückhalteelement 80 kommt bevorzugt zum Einsatz, wenn das Retraktionssystem aus seiner ursprünglich am Körper des Patienten eingenommenen Position heraus geschwenkt werden soll, um so beispielsweise das Präparieren einer Arteria Mammaria zu erleichtern. Hier erleichtert die gelenkige Anbindung des dritten Teils 100 an das zweite Teil den Vorgang, und das Knochengewebe wird dabei schonend von dem Rückhalteelement 80 gehalten.

Das Rückhalteelementelement 80 hat den weiteren Vorteil, dass aufgrund der drei Lagerstellen in einer Ebene senkrecht zur Spreizebene, in die das dritte Teil 100 eingesetzt werden kann, eine Anpassung an die Dicke des Knochenmaterials und des das Knochenmaterial umgebenden sonstigen Gewebematerials vorgenommen werden kann, so dass eine möglichst atraumatische Halterung gewährleistet ist.

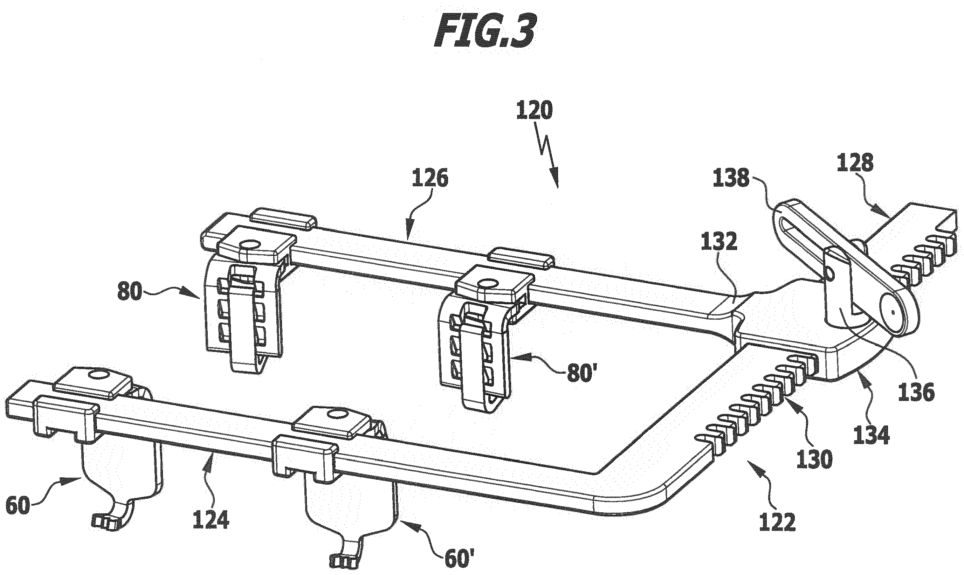

In der Figur 3 ist eine weitere Ausführungsform eines chirurgischen Retraktionssystems 120 gezeigt mit einer Haltevorrichtung 122, an der zwei gerade Spreizarme 124, 126 parallel zueinander angeordnet sind. Die Haltevorrichtung 122 umfasst eine Schiene 128, welche eine an einer Schmalseite ausgebildete Zahnreihe 130 aufweist. An die Haltevorrichtung 122 ist an einem Ende rechtwinklig der erste Spreizarm 124 angeformt.
Sowohl die Haltevorrichtung 122 bzw. deren Schiene 128 als auch die Spreizarme 124 und 126 weisen einen im Wesentlichen rechteckigen Querschnitt auf.
Der Spreizarm 126 weist an einem seiner Enden 132 eine Haltevorrichtung 134 auf, mit der der Spreizarm 126 entlang der Schiene 128 in unterschiedliche Positionen verfahrbar ist. Hierzu weist die Haltevorrichtung 134 eine Antriebsvorrich-tung mit einem Drehkörper (im Einzelnen nicht gezeigt) auf, der beispielsweise die Form eines Zahnrades oder eines Zapfenrades aufweisen kann. Der Drehkörper greift in die Zahnreihe 130 der Schiene 128 ein. Der Drehkörper ist drehfest auf einer Achse 136 montiert, welche wiederum mittels eines Griffteils 138 rotiert werden kann. So lässt sich der Abstand des Spreizarms 126 zu dem Spreizarm 124 verringern oder vergrößern. Der so gestaltete Antrieb für den Spreizarm 126 ist wiederum vorzugsweise selbsthemmend ausgebildet.

Bei dem chirurgischen Retraktionssystem 120 weist der erste Spreizarm 124 zwei Rückhalteelement 60, 60' auf, wie sie im Einzelnen im Zusammenhang mit der Figur 2B beschrieben wurden. An dem Spreizarm 126 sind zwei Rückhalteelement 80, 80' montiert, wie sie im Einzelnen im Zusammenhang mit den Figuren 2C und 2D beschrieben wurden.

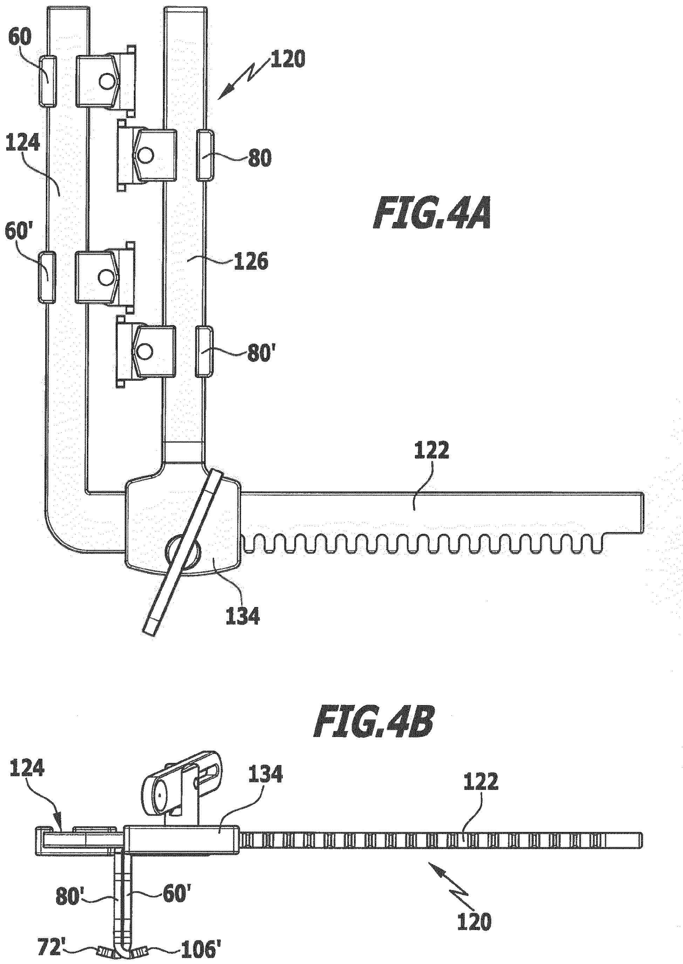

Die Figuren 4A und 4B zeigen eine Draufsicht bzw. eine Seitenansicht auf das chirurgische Retraktionssystem 120 der Figur 3. Aus der Darstellung der Figur 4A ist ersichtlich, dass aufgrund der verschieblichen Lagerung der Rückhalteelemente 60, 60' sowie 80, 80' eine Annäherung der beiden Spreizarme 124 und 126 so weit möglich ist, dass die Rückhalteflächen der Rückhalteelemente praktisch in einer Ebene angeordnet sind. Der dadurch erzielte sehr geringe Platzbedarf parallel zur Spreizebene des Retraktionssystem 120 ermöglicht ein einfaches Einsetzen des Retraktionssystem in den nach einer Durchtrennung des Sternums geschaffenen Spalt in dem Knochenmaterial.

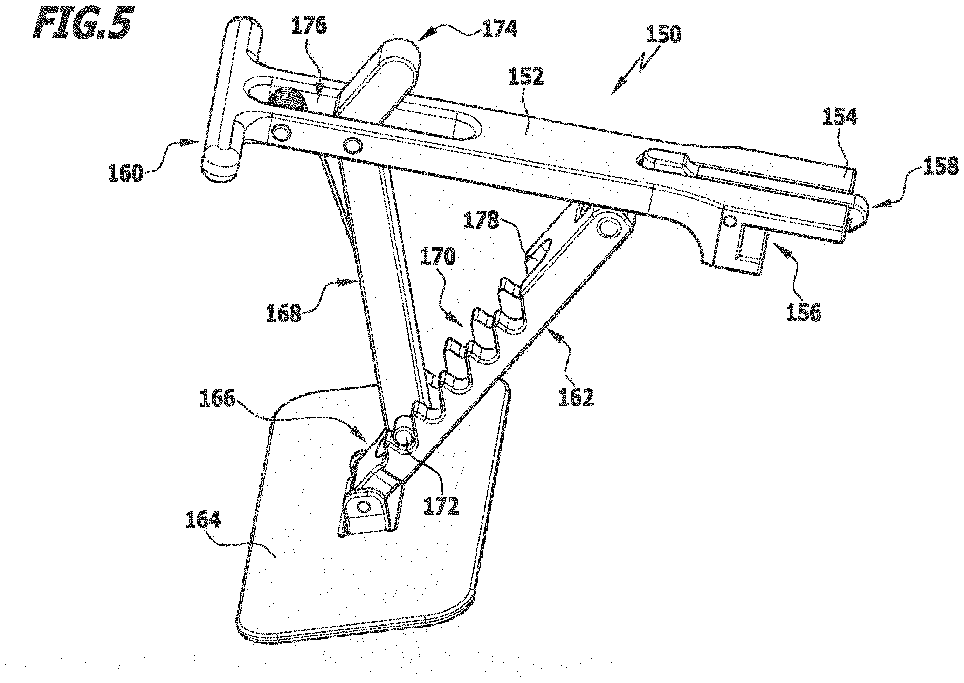

Diese günstige Situation zum Einführen des Retraktionssystems 120 in den Spalt eines geteilten Sternums wird in der Figur 4B in der Seitenansicht nochmals verdeutlicht. Hier wird sichtbar, dass dadurch, dass die Rückhalteflächen der Rückhalteelemente der 60, 60' und 80, 80' sogar leicht gegeneinander versetzt angeordnet sind und der Platzbedarf für das Einführen der am freien Ende der Rückhalteelemente angeordneten Schenkel 106, 106' und 72, 72' minimiert ist.
Bei Herz-Thorax-Operationen muss zur Präparation der Arteria Mammaria häufig eine Seite des von dem chirurgischen Retraktionssystem gehaltenen Knochenmaterials vom Körper des Patienten abgehoben werden, um dem Chirurgen ausreichend Platz für die Präparation zu bieten.
Die Retraktionssysteme, die im Zusammenhang mit den Figuren 1, 3 und 4 bereits beschrieben wurden, lassen sich über eine Zusatzfunktion in Form eines Hebelmechanismus 150, wie er beispielhaft anhand einer bevorzugten Ausführungsform in Figur 5 gezeigt ist, entweder von vornherein oder auch nachträglich, d.h. nach der Applikation des chirurgischen Retraktionssystems und gegebenenfalls im Zustand eines bereits retrahierten Sternums, mit einer Funktion zum Anheben ausrüsten.

Der Hebelmechanismus 150 umfasst ein stangenartiges Halteelement oder Halteteil 152, welches sich mit einem ersten Ende 154, welches als Montageteil ausgebildet ist, an einem Spreizarm drehfest andocken lässt. Hierzu weist das Montageteil 154 einen nutförmigen Abschnitt 156 auf, in den das Rechteckprofil des Spreizarms formschlüssig einrückbar ist. An seiner Oberseite weist das Montageteil 154 einen längsverschiebbaren arretierbaren Haken 158 auf, welcher der Sicherung eines einmal in die Nut 156 eingerückten Spreizarmprofils dient.

An seinem gegenüberliegenden Ende weist das Halteteil 152 ein Griffteil 160 auf, mit welchem sich aufgrund der drehfesten Montage des Halteteils 152 an dem zugehörigen Spreizarm eine Hebelkraft anwenden lässt, mit der der Spreizarm und der daran gehaltene Teil des Sternums angehoben werden kann.

Der Hebelmechanismus 150 weist des Weiteren einen benachbart zum Montageteil 154 gelenkig verbundenen Stangenabschnitt 162 auf, der an seinem entgegengesetzten Ende eine Stützplatte 164, vorzugsweise gelenkig mit einer Schwenkachse parallel zur Spreizebene, hält.

Zur Sicherung einer einmal erreichten Schwenkpositionen des Halteteils 152 und des damit verbundenen Schwenkarms ist ein Abstandhalter 168 vorgesehen, der mit einem Ende benachbart zu dem Griffteil 160 an dem Halteteil 150 schwenkbar gelagert ist und der mit seinem entgegengesetzten Ende in einer wählbaren Position entlang des Stangenabschnitts 162 festgelegt werden kann.

Hierzu weist der Stangenabschnitt 162 bevorzugt ein Zahnstangensegment 170 auf und das freie Ende des Abstandhalters 168 einen an seinem Ende quer angeordneten, zu beiden Seiten überstehenden Stift 172, der in unterschiedliche Positionen des Zahnstangenabschnitts 170 einrückbar ist.

An seinem gelenkig mit dem Halteteil 152 verbundenen Ende weist der Abstandhalter 168 vorzugsweise ein abgekröpftes Bedienelement 174 auf, mit dem beim Ergreifen des Griffteils 160 des Halteelements 152 mit einer Hand auch der Abstandshalter 168 in seiner Schwenkposition verstellt werden kann.

Bevorzugt ist das an dem Halteelement 152 angelenkte Ende des Abstandshalters 168 in einem in dem Halteelement 152 ausgebildeten Langloch 176 schwenkbar gelagert, dessen Wandungen dann eine Führung für den Abstandhalter 168 bei der Schwenkbewegung bilden. Damit ist eine sichere Ausrichtung des Abstandhalters 168 zu dem Zahnstangenabschnitt 170 des Stangenabschnitts 162 gegeben und sichergestellt, dass beim Freigeben des Griffteils 160 der Abstandshalter 168 mit seinem Stift 172 stets in einer Position angeordnet ist, in der der Stift 172 in einen der Rücksprünge des Zahnstangenabschnitts 170 eingreifen kann und damit die Schwenkposition des Halteelements 152 und des damit verbundenen Spreizarms sichert.

Zur weiteren Sicherung des Bewegungsablaufs beim Verschwenken des Halteteils 152 und zur Sicherung des Halteteils 152 in seiner Schwenkposition durch den Abstandhalter 168 und dessen in einen Rücksprung in das Zahnstangensegment 170 eintretenden Stift 170 kann vorgesehen sein, dass der Stangenabschnitt 162 mit einem mittleren Langloch 178 versehen ist, wobei das den Stift 172 tragende Ende des Abstandhalters 168 vorzugsweise durch das Langloch 178 hindurchgreift und auf der dem Zahnstangenabschnitt 170 gegenüberliegenden Seite des Stangenabschnitts 162 gesichert ist. Dies vermeidet, dass das freie Ende des Abstandhalters 168 aus dem Langloch 178 heraustreten kann. So ist immer gewährleistet, dass der Stift 172 in einen der Rücksprünge des Zahnstangensegments 170 eingreifend platziert werden kann.

Wird das freie Ende des Abstandhalters 168 in seiner Position in dem Langloch 178 nicht oder nicht permanent gesichert, dann besteht die Möglichkeit, den Abstandhalter 168 mit seinem den Stift 172 tragenden Ende von dem Zahnstangensegment 170 des Abstandhalters 168 zu lösen und im Wesentlichen parallel zu dem Halteteil 152 zu verschwenken, wodurch sich der Hebelmechanismus 150 platzsparend zusammenfalten lässt. Dies ist insbesondere dann wünschenswert, wenn der Hebelmechanismus 150 erst nachdem das erfindungsgemäße chirurgische Retraktionssystem in dem geteilten Sternum platziert und die Retraktion vorgenommen wurde an dem System befestigt wird, wobei der Hebelmechanismus 150 dann ohne Zwänge an dem Spreizarm befestigbar ist.

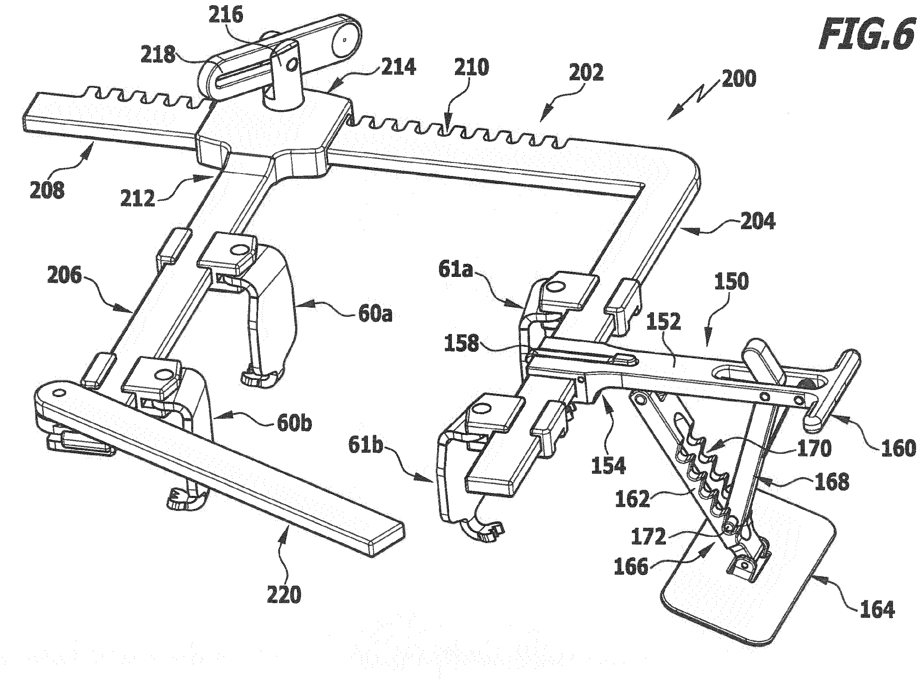

In der Figur 6 ist ein chirurgisches Retraktionssystem 200 mit einer Haltevorrichtung 202, an der zwei gerade Spreizarme 204, 206 parallel zueinander angeordnet sind, gezeigt. Die Haltevorrichtung 202 umfasst eine Schiene 208, welche eine an einer Schmalseite ausgebildete Zahnreihe 210 aufweist. An der Haltevorrichtung 202 ist an einem Ende rechtwinklig der erste Spreizarm 204 angeformt.

Sowohl die Haltevorrichtung 202 bzw. deren Schiene 208 als auch die Spreizarme 204 und 206 weisen einen im Wesentlichen rechteckigen Querschnitt auf.

Der Spreizarm 206 ist ebenfalls im Wesentlichen mit einem rechteckigen Querschnitt ausgebildet und weist an einem seiner Enden 212 eine Haltevorrichtung 214 auf, mit der der Spreizarm 206 entlang der Schiene 208 in unterschiedliche Positionen verfahrbar ist. Hierzu weist die Haltevorrichtung 214 einen Antriebsmechanismus mit einem Drehkörper (im Einzelnen nicht gezeigt) auf, der beispielsweise die Form eines Zahnrades oder eines Zapfenrades aufweisen kann. Der Drehkörper greift in die Zahnreihe 210 der Schiene 208 ein. Der Drehkörper ist drehfest auf einer Achse 216 montiert, welche wiederum mittels eines Griffteils 218 rotiert werden kann. So lässt sich der Abstand des Spreizarms 206 zu dem Spreizarm 204 verringern oder vergrößern. Der Antriebsmechanismus ist vorzugsweise wieder selbsthemmend ausgeführt.

Bei dem chirurgischen Retraktionssystem 200 weist der erste Spreizarm 204 zwei Rückhalteelemente 60a, 60b auf; an dem Spreizarm 206 sind zwei Rückhalteelemente 61a, 61b montiert. Die Rückhalteelemente 60a, 60b und 61a, 61b sind vom Typ des Rückhalteelements 60, wie es im Einzelnen im Zusammenhang mit der Figur 2B beschrieben wurde.

An dem mit der Haltevorrichtung 202 einstückig ausgebildeten Spreizarm 204 ist ein Hebelmechanismus 150, wie er im Zusammenhang mit der Figur 5 beschrieben wurde, montiert. Wegen der Einzelheiten des Hebelmechanismus 150 darf deshalb auf die Beschreibung der Figur 5 Bezug genommen werden.

Der Abstandhalter 168 ist mit seinem freien Ende 166 in der untersten Raststellung des Zahnsegments 170 gehalten, entsprechend einem geringsten Grad der Verschwenkung bzw. des einseitigen Anhebens, in dem das Retraktionssystems 200 fixiert werden kann. Wird der Hebelarm 152 mit dem Griffteil 160 weiter angehoben, kann der Abstandhalter 168 in höheren Raststellungen des Zahnsegments 170 fixiert und so dem Chirurgen, falls erforderlich, ein weiter verbesserter Zugang zur Arteria Mammaria geschaffen werden.

Am freien Ende des Spreizarms 206 ist vorzugsweise lösbar ein parallel zur Spreizebene verschwenkbar gehaltener Montagebügel 220 gehalten, an dem bei Bedarf weiteres Zubehör, wie z.B. Beleuchtungsmittel, Fadenhaltebänkchen, Haltearme mit Niederhalter etc., positioniert werden können.

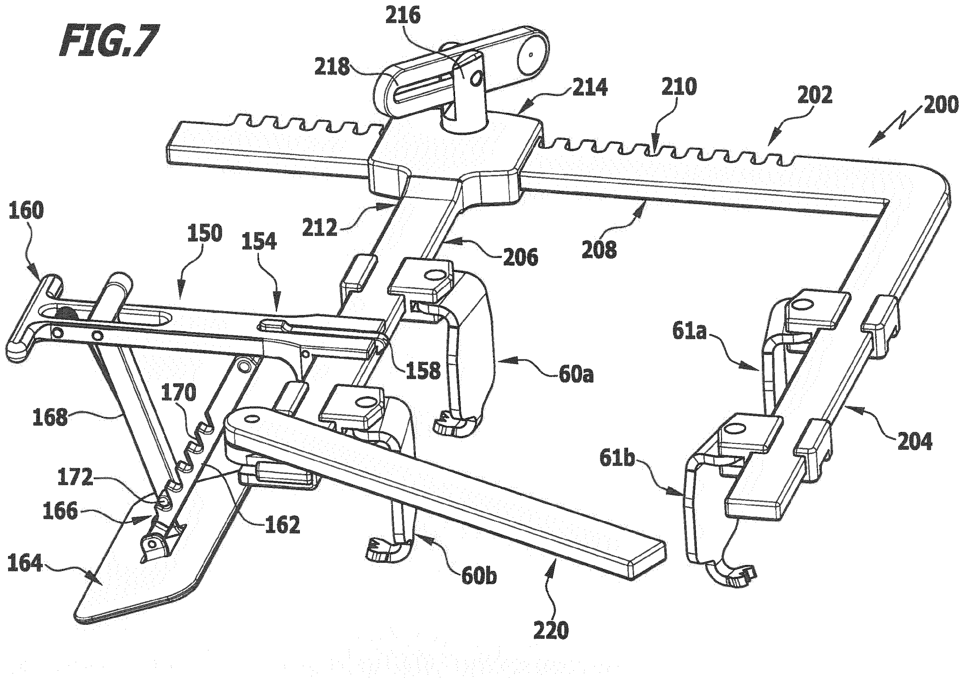

Figur 7 zeigt das chirurgische Retraktionssystem 200 der Figur 6, jedoch ist in dieser Darstellung der Hebelmechanismus 150 an dem an der Haltevorrichtung 202 verschieblich positionierbar gehaltenen Spreizarm 206 fixiert. Der Chirurg hat jetzt die Möglichkeit, die andere Arteria Mammaria zu präparieren.

Das chirurgische Retraktionssystem erlaubt es, den Hebelmechanismus 150 einfach durch Lösen des Schiebers 158 von einem der Spreizarme abzunehmen und an dem anderen Spreizarm zu fixieren und so ohne Entfernen des Spreizarme abzunehmen und an dem anderen Spreizarm zu fixieren und so ohne Entfernen des Retraktionssystems und auch ohne Änderung in der Retraktion der Sternumhälften die andere Seite des Sternums anzuheben und so die weitere Arteria Mammaria zur Präparation zugänglich zu machen. Dies ist nicht nur mit einer erheblichen Zeitersparnis im Operationsablauf verbunden, sondern auch mit deutlich geringeren traumatischen Folgen für den Patienten.

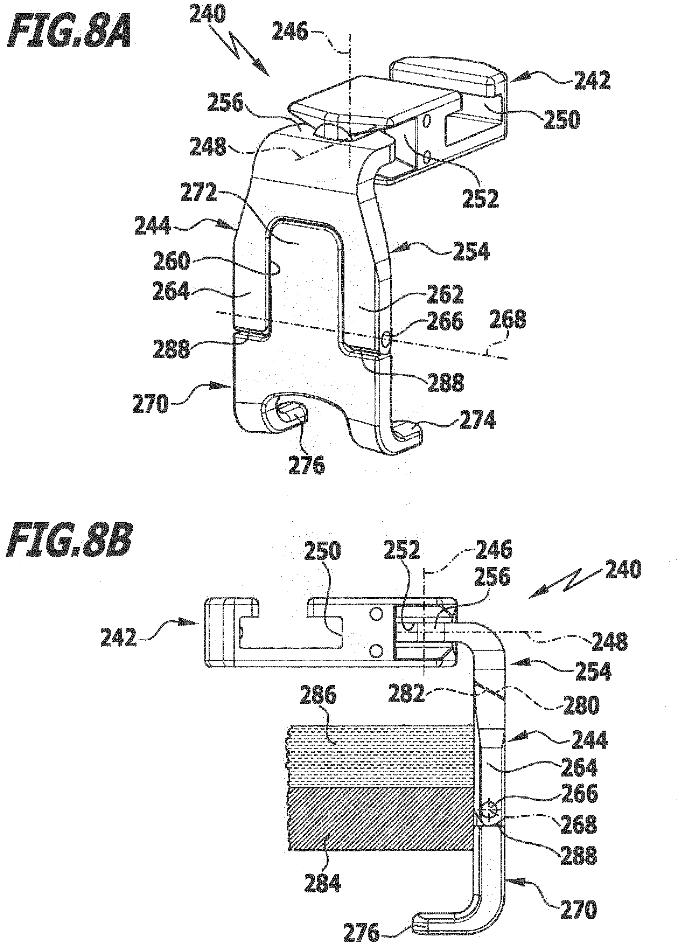

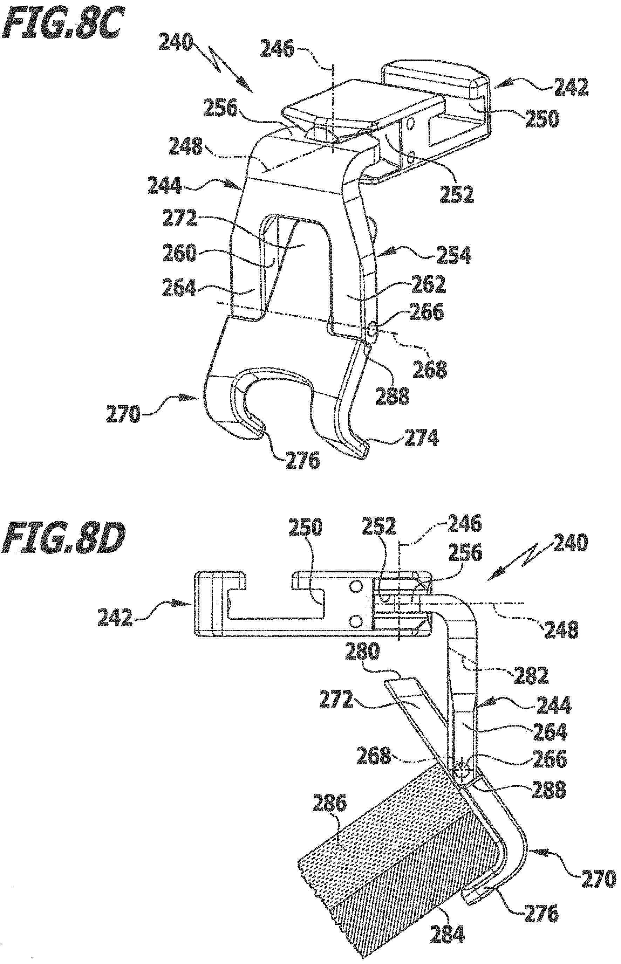

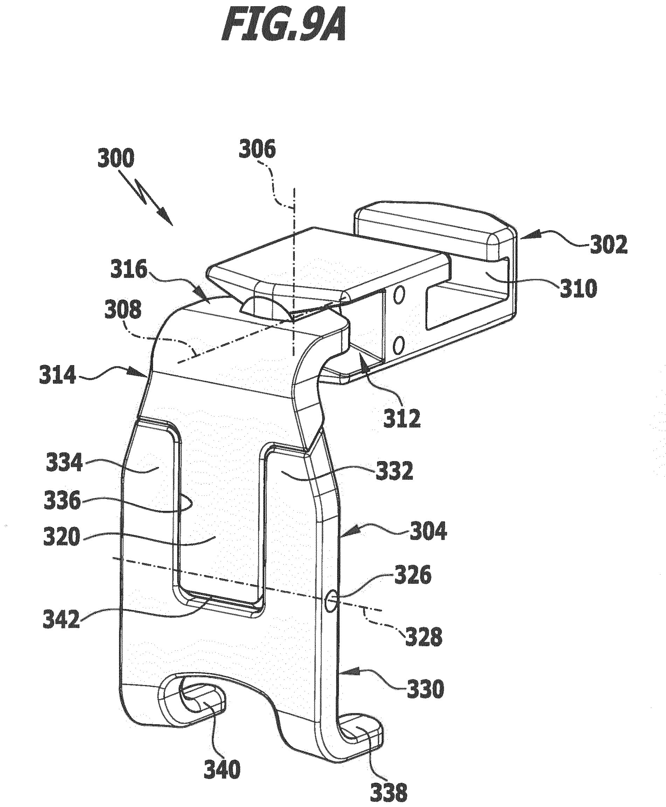

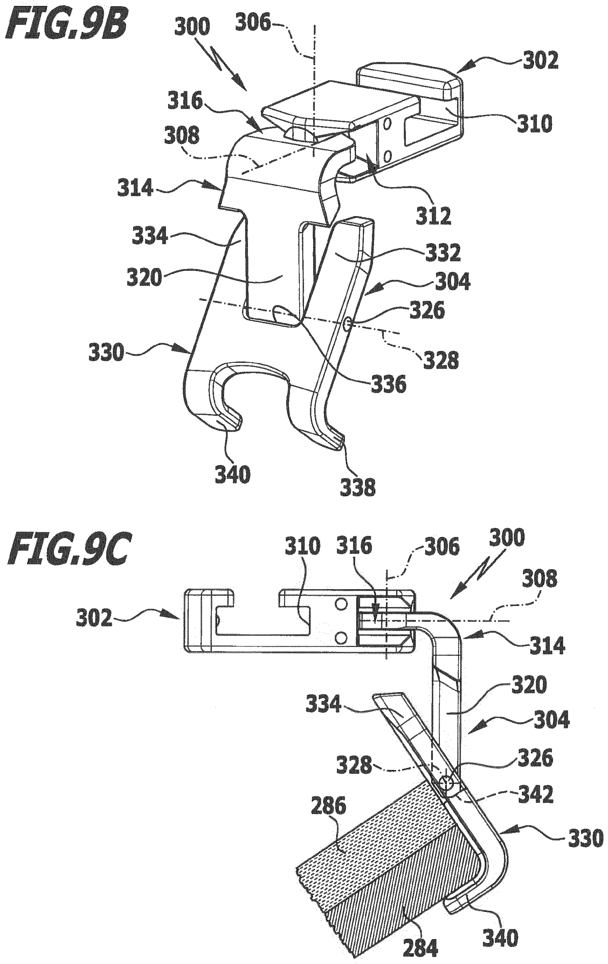

In den Figuren 8 und 9 sind weitere Varianten von Rückhalteelementen, die im Zusammenhang mit dem erfindungsgemäßen chirurgischen Retraktionssystem verwendet werden können, in verschiedenen Stellungen und Perspektiven gezeigt.

Die Figuren 8A bis 8D zeigen ein Rückhalteelement 240, das mehrteilig ausgebildet ist, wobei ein erstes Teil als Halteteil oder Lagerkörper 242 der Montage des Rückhalteelements 240 an dem jeweils zugeordneten Spreizarm dient. Ein zweiter Teil 244 bildet eine Rückhaltefläche, an der zu retrahierendes Knochenmaterial bzw. daran angrenzendes Weichgewebe in Anlage gebracht werden kann. Die Rückhaltefläche 244 ist an dem Lagerkörper 242 um eine erste, senkrecht zur Spreizebene angeordnete Achse 246 drehbar gelagert.

Darüber hinaus ist bei dem Rückhalteelement 240 vorgesehen, dass die Rückhaltefläche 244 um eine zweite Achse schwenkbar an dem Lagerkörper 242 gehalten ist, wobei die zweite Achse 248 senkrecht zur ersten Achse 246 sowie parallel zur Spreizebene angeordnet ist.

Der Lagerkörper 242 des Rückhalteelements 240 weist in einem ersten Bereich ein im Wesentlichen rechteckiges, nach oben offenes Hohlprofil 250 auf, das im Wesentlichen einem Querschnitt eines zugeordneten Spreizarms entspricht und diesen entsprechend gleitend aufnehmen kann. An einer der Schmalseiten weist der Lagerkörper 242 einen sich lateral öffnenden C-förmigen Querschnittsbereich 252 auf, in dem die Doppelgelenkverbindung mit den Schwenkachsen 246, 248 zur Halterung der Rückhaltefläche 244 ausgebildet ist.

Die Rückhaltefläche 244 ist in zwei getrennte Bereiche untergliedert, wobei ein erster Bereich 254 mit einem im Wesentlichen rechtwinklig abgekröpften Abschnitt 256 in den C-förmigen Querschnittsbereich 252 eingreift und dort über die beiden Schwenkachsen 246, 248 gelagert ist.

Das von dem Lagerkörper 242 weg weisende Ende des ersten Bereichs 254 der Rückhaltefläche 244 ist gabelförmig ausgebildet mit einer mittigen Ausnehmung 260 und den beiden zinkenförmigen Vorsprüngen 262, 264. An den freien Enden der beiden Vorsprünge 262, 264 ist ein Achslager 266 ausgebildet, welches eine Drehachse 268 definiert, die einen zweiten Bereich 270 der Rückhaltefläche 244 lagert.

Der zweite Bereich 270 der Rückhaltefläche 244 weist auf einer Seite einen Vorsprung 272 auf, welcher im Wesentlichen komplementär zu der Ausnehmung 260 des ersten Bereichs 254 ausgebildet ist.

Im Bereich des Vorsprungs 272 ist dann ein Lager für die Drehachse 268 vorgesehen, über die der zweite Bereich 270 am ersten Bereich 244 schwenkbar gelagert ist.

Am freien Ende des zweiten Bereichs 270 sind zwei abgekröpfte stegartige Vorsprünge 274, 276 vorgesehen, welche der Sicherung des von dem Rückhalteelement 240 erfassten Weichgewebes 286 und Knochenmaterials 284 bei einer Retraktion dienen.

Bevorzugt ist der in die Ausnehmung 260 hineinragende Teil des Vorsprungs 272 des zweiten Bereichs 270 des Rückhalteelements 240 mit einer Fase 280 versehen, die komplementär zu einem angefasten Bereich 282 der Ausnehmung 216 ausgebildet ist, so dass dieser einen Anschlag für den Vorsprung 272 des zweiten Bereichs 270 bildet und damit eine mögliche Schwenkbewegung des zweiten Bereichs 270 gegenüber dem ersten Bereich 254 der Rückhaltefläche 244 begrenzt.
Dies vereinfacht insbesondere die Handhabung der Rückhalteelemente beim Einsetzen der damit ausgerüsteten Retraktionssysteme in ein einmal geteiltes Sternum.
In den Figuren 8A und 8B ist das Rückhalteelement 240 in der Neutralposition gezeigt, in der das Retraktionssystem in das geteilte Sternum zunächst eingesetzt wird. In dieser Stellung bleibt das Rückhalteelement 240 insbesondere auch mit seinem zweiten Bereich 270 im Wesentlichen erhalten, auch wenn Retraktionskräfte ausgeübt werden.

Wird dann das Retraktionssystem, wie im Zusammenhang mit den Figuren 6 und 7 beschrieben, mittels eines Hebelmechanismus aus der zunächst gegebenen Retraktionsebene heraus verschwenkt, dann ermöglicht die schwenkbare Lagerung des zweiten Bereichs 270 des Rückhalteelements 240 eine weiterhin möglichst großflächige Anlage des Rückhalteelements 240 an dem Weichgewebe 286 und dem Knochenmaterial 284 des Sternums, wie in den Figuren 8C und insbesondere 8D schematisch gezeigt ist.
Am unteren Ende des ersten Bereichs 254 der Rückhaltefläche 244 ist eine weitere Fase 288 vorgesehen, die einen weiteren Anschlag für den zweiten Bereich 270 darstellt. Mit dieser Fase 288 kann der Winkel, um welchen der Bereich 270 mittels der Drehachse 268 schwenkt, begrenzt werden, so dass der zweite Bereich 270 nicht so weit verkippen kann, dass das Weichgewebe 286 und das Knochenmaterial 284 des Sternums sich vom Vorsprung 276 lösen und herausrutschen könnte. In den Figuren 8C und 8D ist der zweite Bereich in einer maximal verschwenkten Stellung gezeigt.

Die Figuren 9A bis 9C zeigen ein Rückhalteelement 300, das ebenfalls mehrteilig ausgebildet ist, wobei ein erstes Teil als Halteteil oder Lagerkörper 302 der Montage des Rückhalteelements 300 an dem jeweils zugeordneten Spreizarm dient. Ein zweiter Teil 304 bildet eine Rückhaltefläche, an der zu retrahierendes Knochenmaterial 284 des Sternums bzw. daran angrenzendes Weichgewebe 286 in Anlage gebracht werden kann. Die Rückhaltefläche 304 ist an dem Lagerkörper 302 um eine erste, senkrecht zur Spreizebene angeordnete Achse 306 drehbar gelagert.

Darüber hinaus ist auch bei dem Rückhalteelement 300 vorgesehen, dass die Rückhaltefläche 304 um eine zweite Achse schwenkbar an dem Lagerkörper 302 gehalten ist, wobei die zweite Achse 308 senkrecht zur ersten Achse 306 sowie parallel zur Spreizebene angeordnet ist.

Der Lagerkörper 302 des Rückhalteelements 300 weist in einem ersten Bereich ein im Wesentlichen rechteckiges, nach oben offenes Hohlprofil 310 auf, das im Wesentlichen einem Querschnitt eines zugeordneten Spreizarms entspricht und diesen entsprechend gleitend aufnehmen kann. An einer der Schmalseiten weist der Lagerkörper 302 einen sich lateral öffnenden C-förmigen Querschnittsbereich 312 auf, in dem die Doppelgelenkverbindung mit den Schwenkachsen 306, 308 zur Halterung der Rückhaltefläche 304 ausgebildet ist.

Die Rückhaltefläche 304 ist in zwei getrennte Bereiche untergliedert, wobei ein erster Bereich 314 mit einem im Wesentlichen rechtwinklig abgekröpften Abschnitt 316 in den C-förmigen Querschnittsbereich 312 eingreift und dort über die beiden Schwenkachsen 306, 308 gelagert ist.

Das von dem Lagerkörper 302 weg weisende Ende des ersten Bereichs 314 der Rückhaltefläche 304 ist mit einem mittigen Vorsprung 320 ausgebildet. Am freien Ende des Vorsprungs 320 ist ein Achslager 326 ausgebildet, welches eine Drehachse 328 definiert, die einen zweiten Bereich 330 der Rückhaltefläche 304 lagert.

Der zweite Bereich 330 der Rückhaltefläche 304 ist auf einer Seite gabelförmig mit zwei Vorsprüngen 332, 334 ausgebildet, zwischen denen im Wesentlichen komplementär zu dem Vorsprung 320 des ersten Bereichs 314 eine Ausnehmung 336 ausgebildet ist.

Im Bereich der Vorsprünge 332, 334 ist dann ein Lager für die Drehachse 328 vorgesehen, über die der zweite Bereich 330 an dem ersten Bereich 314 schwenkbar gelagert ist.

Am freien Ende des zweiten Bereichs 330 sind zwei abgekröpfte stegartige Vorsprünge 338, 340 vorgesehen, welche der Sicherung des von dem Rückhalteelement 300 erfassten Weichgewebes 286 und Knochenmaterials 284 des Sternums bei einer Retraktion dienen.

Bevorzugt ist der in die Ausnehmung 336 hineinragende Teil des Vorsprungs 320 des ersten Bereichs 314 des Rückhalteelements 300 mit einer Fase versehen, die komplementär zu einem angefasten Bereich der Ausnehmung 336 ausgebildet ist, so dass dieser einen Anschlag für den Vorsprung 320 des ersten Bereichs 314 bildet und damit eine mögliche Schwenkbewegung des zweiten Bereichs 270 gegenüber dem ersten Bereich 314 der Rückhaltefläche 304 begrenzt.

Dies vereinfacht insbesondere die Handhabung der Rückhalteelemente beim Einsetzen der damit ausgerüsteten Retraktionssysteme in ein einmal geteiltes Sternum.

In der Figur 9A ist das Rückhalteelement 300 in der Neutralposition gezeigt, in der das Retraktionssystem in das geteilte Sternum zunächst eingesetzt wird. In dieser Stellung bleibt das Rückhalteelement 300 insbesondere auch mit seinem zweiten Bereich 330 im Wesentlichen erhalten, auch wenn Retraktionskräfte ausgeübt werden.

Wird dann das Retraktionssystem, wie im Zusammenhang mit den Figuren 6 und 7 beschrieben, mittels eines Hebelmechanismus aus der zunächst gegebenen Retraktionsebene heraus verschwenkt, dann ermöglicht die schwenkbare Lagerung des zweiten Bereichs 330 des Rückhalteelements 300 eine weiterhin möglichst großflächige Anlage des Rückhalteelements 300 an dem Weichgewebe 286 und dem Knochenmaterial 284 des Sternums, wie in den Figuren 9B und insbesondere 9C schematisch gezeigt ist.
Auch bei der Ausführungsform des Rückhalteelements 300 empfiehlt sich die Ausbildung eines weiteren Anschlags 342 am ersten Bereich 314 der Rückhaltefläche 304, so dass ein Verschwenken des zweiten Bereichs 330 aus der Neutralstellung (Fig. 9A) heraus ebenfalls, wie in den Figuren 9B und 9C gezeigt, begrenzbar ist. Auch hier wird dann ein unbeabsichtigtes Lösen der Vorsprünge 338 und 340 von dem Sterum 284 und dem angrenzenden Weichgewebe 286 vermieden.

## Patentansprüche

1. Chirurgisches Retraktionssystem (10), insbesondere zum Spreizen eines durchtrennten Sternums, mit einer Haltevorrichtung (12), zwei Spreizarmen (14, 16), welchletztere jeweils mit einem ersten Ende an der Haltevorrichtung (12) in einem in einer Spreizebene veränderlichen Abstand voneinander gehalten sind, sowie mindestens zwei Rückhalteelementen (240), wobei an jedem der Spreizarme (14, 16) jeweils mindestens ein Rückhalteelement (240) angeordnet ist, wobei die Rückhalteelemente (240) mehrteilig ausgebildet sind, wobei ein erstes Teil der Rückhalteelemente (240) einen Lagerkörper (242) bildet, der am Spreizarm (14, 16) gehalten ist, wobei an dem ersten Teil ein zweites Teil des Rückhalteelements (240) in Form einer Rückhaltefläche (244) um eine im Wesentlichen senkrecht zur Spreizebene des Retraktionssystems (10) ausgerichtete, erste Achse (246) drehbar gehalten ist, und wobei der Lagerkörper (242) von mindestens einem der Rückhalteelemente (240) die Rückhaltefläche (244) um eine zweite Achse (248) schwenkbar hält, wobei die zweite Achse (248) im Wesentlichen senkrecht zur ersten Achse (246) und zur Rückhaltefläche (244) ausgerichtet ist,
**dadurch gekennzeichnet, dass** die Rückhaltefläche (244) von mindestens einem der Rückhalteelemente (240) in zwei Bereiche (254, 270) geteilt ausgebildet ist, wobei der erste Bereich (254) der Rückhaltefläche (244) an dem Lagerkörper (242) gehalten ist und wobei der zweite Bereich (270) an dem ersten Bereich (254) um eine dritte Achse (268) schwenkbar gehalten ist, wobei die dritte Achse (268) im Wesentlichen parallel zur Rückhaltefläche (244) und im Wesentlichen senkrecht zur ersten Achse (246) ausgerichtet ist, und dass der erste Bereich (254) eine Ausnehmung (260) aufweist, welche sich vom Bereich der dritten Achse (268) in Richtung zum Lagerkörper (242) erstreckt, und
dass der zweite Bereich (270) einen zur Ausnehmung (260) des ersten Bereichs im Wesentlichen komplementären Vorsprung (272) umfasst, wobei vorzugsweise am ersten Bereich (254) ein Anschlag (282) ausgebildet ist, an dem der zweite Bereich (270) zur Anlage bringbar ist, oder
dass der erste Bereich (314) einen Vorsprung (320) aufweist, welcher an seinem freien Ende ein Lager für die dritte Achse (326) umfasst, und dass der zweite Bereich (330) eine zu dem Vorsprung (320) des ersten Bereichs (384) im Wesentlichen komplementäre Ausnehmung aufweist, wobei vorzugsweise am ersten Bereich (314) ein Anschlag ausgebildet ist, an dem der zweite Bereich (330) zur Anlage bringbar ist.

2. Chirurgisches Retraktionssystem (10) nach Anspruch 1, **dadurch gekennzeichnet, dass** die Rückhaltefläche von mindestens einem der Rückhalteelemente (80) in zwei Bereiche (90, 100) geteilt ausgebildet ist, wobei der erste Bereich (90) der Rückhaltefläche an dem Lagerkörper (84) gehalten ist, wobei der zweite Bereich (100) gegenüber dem ersten Bereich (90) in einer Ebene parallel zur ersten Achse bewegbar gelagert ist, wobei insbesondere der zweite Bereich (100) in unterschiedlichen Abständen zum Lagerteil des Rückhalteelements entlang der ersten Achse positionierbar ist.

3. Chirurgisches Retraktionssystem nach einem der Ansprüche 1 oder 2, **dadurch gekennzeichnet, dass** die Rückhaltefläche (244) mindestens eines der Rückhalteelemente (240) zum ersten Teil hin trapezförmig verjüngt ausgebildet ist.

4. Chirurgisches Retraktionssystem nach einem der Ansprüche 1 bis 3, **dadurch gekennzeichnet, dass** die Rückhaltefläche (68) mindestens eines der Rückhalteelemente (60) an ihrem freien, zum Spreizarm (14, 16) entfernt gelegenen Ende eine geringere Ausdehnung senkrecht zur ersten Achse aufweist als in einem davon entfernten Bereich,
wobei optional das freie, zum Spreizarm (14, 16) entfernt gelegene Ende der Rückhaltefläche zu einem stegartigen Vorsprung (68) verjüngt ausgebildet ist.

5. Chirurgisches Retraktionssystem nach Anspruch 4, **dadurch gekennzeichnet, dass** das freie, zum Spreizarm (14, 16) entfernt gelegene Ende der Rückhaltefläche einen abgewinkelten Abschnitt (72) umfasst.

6. Chirurgisches Retraktionssystem nach einem der Ansprüche 1 bis 5, **dadurch gekennzeichnet, dass** an jedem Spreizarm (14, 16) mindestens zwei Rückhalteelemente (240) angeordnet sind,
wobei optional die Rückhalteelemente (240) der beiden Spreizarme (14, 16) in Längsrichtung der Spreizarme (14, 16) gesehen versetzt zueinander angeordnet sind.

7. Chirurgisches Retraktionssystem nach einem der Ansprüche 1 bis 6, **dadurch gekennzeichnet, dass** das oder die Rückhalteelemente (240) in ihrem Abstand zur Haltevorrichtung (12) und vorzugsweise auch zueinander entlang des Spreizarms (14, 16) verstellbar sind.

8. Chirurgisches Retraktionssystem (200) nach einem der Ansprüche 1 bis 7, **dadurch gekennzeichnet, dass** das System (200) ferner eine Hebevorrichtung (150) aufweist, wobei die Hebevorrichtung (150) ein sich im Wesentlichen parallel zur Spreizebene des Systems erstreckendes Halteelement (152), welches mit einem ersten Ende an einem der Spreizarme vorzugsweise lösbar gehalten ist, eine sich vom Haltelement (152) aus erstreckende Stützvorrichtung (162, 168) und eine am freien Ende der Stützvorrichtung (162, 168) gehaltene Stützplatte (164) zur Anlage am Brustkorb des Patienten umfasst, wobei die Stützvorrichtung (162, 168) die Stützplatte (164) in einem variablen, vorgebbaren Abstand zur Spreizebene hält.

9. Chirurgisches Retraktionssystem (200) nach Anspruch 8, **dadurch gekennzeichnet, dass** die Stützvorrichtung (162, 168) eine Schwenkvorrichtung mit einem Schwenkarm (162) und einen Abstandhalter (168) umfasst, wobei der Schwenkarm (162) mit einem ersten Ende gelenkig an dem Halteelement (152) der Hebevorrichtung (150), benachbart zum Spreizarm montiert ist und an seinem entgegengesetzten, zweiten Ende das Stützelement trägt und wobei der Abstandhalter (168) mit einem ersten Ende benachbart zum zweiten Ende des Halteelements (152) an diesem gehalten ist und sich mit seinem zweiten Ende an dem Schwenkarm (162) abstützt und so das das Stützelement tragende zweite Ende des Schwenkarms (162) in einem variabel vorgebbaren Abstand zum zweiten Ende des Halteelement hält,
wobei optional der Abstandhalter (168) längenveränderlich ausgebildet ist,
oder alternativ der Abstandhalter (168) mit einem ersten Ende benachbart zu dem zweiten Ende des Halteelements (152) an diesem gelenkig gehalten ist, wobei an dem Schwenkarm (162) mehrere Raststellungen ausgebildet sind, in denen das zweite Ende des Abstandhalters (168) lösbar positionierbar ist,
oder alternativ der Abstandhalter (168) mit einem ersten Ende benachbart zum zweiten Ende des Schwenkarms gelenkig gehalten ist, wobei an dem Halteelement (152) mehrere Raststellungen ausgebildet sind, in denen das zweite Ende des Abstandhalters lösbar positionierbar ist.

10. Chirurgisches Retraktionssystem nach Anspruch 9, **dadurch gekennzeichnet, dass** die Raststellungen als Zahnstangensegment (170) ausgebildet sind.

11. Chirurgisches Retraktionssystem nach Anspruch 9 oder 10, **dadurch gekennzeichnet, dass** das Halteelement (152) an seinem zweiten Ende ein Griffelement (160) aufweist.

12. Chirurgisches Retraktionssystem nach einem der Ansprüche 9 bis 11, **dadurch gekennzeichnet, dass** das Halteelement (152) benachbart zu seinem zweiten Ende eine Langlochöffnung (176) aufweist, in der der Abstandhalter (168) mit seinem ersten Ende verschwenkbar gehalten ist, wobei optional der Abstandhalter (168) an seinem ersten Ende ein Griffteil (174) aufweist, welches durch die Langlochöffnung (176) des Halteelements (152) hindurchreicht.

## Claims

1. Surgical retraction system (10), in particular for spreading a severed sternum, with a holding apparatus (12), two spreading arms (14, 16) which are each held with a first end on the holding apparatus (12) at a variable spacing from each other in a spreading plane, and at least two retaining elements (240), wherein in each case at least one retaining element (240) is arranged on each of the spreading arms (14, 16), wherein the retaining elements (240) are of multi-part configuration, wherein a first part of the retaining elements (240) forms a bearing body (242) which is held on the spreading arm (14, 16), wherein a second part of the retaining element (240) in the form of a retaining face (244) is held on the first part so as to be rotatable about a first axis (246) aligned substantially perpendicular to the spreading plane of the retraction system (10), and wherein the bearing body (242) of at least one of the retaining elements (240) holds the retaining face (244) so as to be pivotable about a second axis (248), wherein the second axis (248) is aligned substantially perpendicular to the first axis (246) and to the retaining face (244), **characterized in that**
the retaining face (244) of at least one of the retaining elements (240) is configured divided into two regions (254, 270), wherein
the first region (254) of the retaining face (244) is held on the bearing body (242) and wherein the second region (270) is held on the first region (254) so as to be pivotable about a third axis (268), wherein the third axis (268) is aligned substantially parallel to the retaining face (244) and substantially perpendicular to the first axis (246), and **in that** the first region (254) has a recess (260) which extends from the region of the third axis (268) in the direction to the bearing body (242), and
**in that** the second region (270) comprises a projection (272) substantially complementary to the recess (260) of the first region, wherein at the first region (254) is preferably formed a stop (282) against which the second region (270) is able to be brought into abutment,
or
**in that** the first region (314) has a projection (320) which comprises at its free end a bearing for the third axis (326), and **in that** the second region (330) has a recess substantially complementary to the projection (320) of the first region (384), wherein at the first region (314) is preferably formed a stop against which the second region (330) is able to be brought into abutment.

2. Surgical retraction system (10) in accordance with Claim 1, **characterized in that** the retaining face of at least one of the retaining elements (80) is configured divided into two regions (90, 100), wherein the first region (90) of the retaining face is held on the bearing body (84), wherein the second region (100) is mounted so as to be moveable relative to the first region (90) in a plane parallel to the first axis, wherein in particular the second region (100) is positionable along the first axis at different spacings from the bearing part of the retaining element.

3. Surgical retraction system in accordance with any one of Claims 1 or 2, **characterized in that** the retaining face (244) of at least one of the retaining elements (240) is configured trapezoidally tapered toward the first part.

4. Surgical retraction system in accordance with any one of Claims 1 to 3, **characterized in that** the retaining face (68) of at least one of the retaining elements (60) has at its free end remote from the spreading arm (14, 16) a smaller extent perpendicular to the first axis than in a region remote therefrom,
wherein optionally the free end of the retaining face located remote from the spreading arm (14, 16) is configured tapered to a weblike projection (68).

5. Surgical retraction system in accordance with Claim 4, **characterized in that** the free end of the retaining face located remote from the spreading arm (14, 16) comprises an angled section (72).

6. Surgical retraction system in accordance with any one of Claims 1 to 5, **characterized in that** at least two retaining elements (240) are arranged on each spreading arm (14, 16),
wherein optionally the retaining elements (240) of the two spreading arms (14, 16) are arranged offset from each other seen in longitudinal direction of the spreading arms (14, 16).

7. Surgical retraction system in accordance with any one of Claims 1 to 6, **characterized in that** the retaining element (240) or retaining elements (240) are adjustable along the spreading arm (14, 16) in their spacing from the holding apparatus (120) and preferably also from each other.

8. Surgical retraction system (200) in accordance with any one of Claims 1 to 7, **characterized in that** the system (200) further has a lifting apparatus (150), wherein the lifting apparatus (150) comprises a holding element (152) which extends substantially parallel to the spreading plane of the system and which is preferably detachably held to one of the spreading arms with a first end, a support apparatus (162, 168) extending from the holding element (152), and a support plate (164) held on the free end of the support apparatus (162, 168) for abutting against the ribcage of the patient, wherein the support device (162, 168) holds the support plate (164) at a variable, specifiable spacing from the spreading plane.

9. Surgical retraction system (200) in accordance with Claim 8, **characterized in that** the support apparatus (162, 168) comprises a pivot apparatus with a pivoting arm (162) and a spacer (168), wherein the pivoting arm (162) is articulatedly mounted with a first end on the holding element (152) of the lifting apparatus (150), adjacent to the spreading arm, and bears the support element on its opposite second end, and wherein the spacer (168) is held on the holding element (152) with its first end adjacent to the second end thereof and is supported on the pivoting arm (162) with its second end and thus holds the second end of the pivoting arm (162) bearing the support element at a variably specifiable spacing from the second end of the holding element, wherein optionally the spacer (168) is configured to be longitudinally adjustable,
or alternatively the spacer (168) is articulatedly held on the holding element (152) with a first end adjacent to the second end thereof, wherein on the pivoting arm (162) are formed multiple latching positions in which the second end of the spacer (168) is releasably positionable, or alternatively the spacer (168) is articulatedly held with a first end adjacent to the second end of the pivoting arm, wherein on the holding element (152) are formed multiple latching positions in which the second end of the spacer is releasably positionable.

10. Surgical retraction system in accordance with Claim 9, **characterized in that** the latching positions are configured as a toothed rack segment (170).

11. Surgical retraction system in accordance with Claim 9 or 10, **characterized in that** the holding element (152) has a grip element (160) at its second end.

12. Surgical retraction system in accordance with any one of Claims 9 to 11, **characterized in that** the holding element (152) has adjacent to its second end an elongated hole in which the spacer (168) is pivotably held with its first end, wherein optionally the spacer (168) has at its first end a grip part (174) which reaches through the elongated hole (176) of the holding element (152).

## Revendications

1. Système de rétraction chirurgical (10), destiné en particulier à écarter un sternum incisé, comprenant un dispositif de maintien (12), deux bras écarteurs (14, 16), lesquels sont maintenus respectivement par une première extrémité sur le dispositif de maintien (12) à distance l'un de l'autre, laquelle distance varie dans un plan d'écartement, ainsi qu'au moins deux éléments de retenue (240), dans lequel respectivement au moins un élément de retenue (240) est agencé sur chacun des bras écarteurs (14, 16), dans lequel les éléments de retenue (240) sont réalisés en plusieurs parties, dans lequel une première partie des éléments de retenue (240) forme un corps support (242) qui est maintenu sur le bras écarteur (14, 16), dans lequel une deuxième partie de l'élément de retenue (240) prenant la forme d'une surface de retenue (244) est maintenue sur la première partie de manière à pouvoir tourner autour d'un premier axe (246) orienté sensiblement perpendiculairement au plan d'écartement du système de rétraction (10), et dans lequel le corps support (242) d'au moins un des éléments de retenue (240) maintient la surface de retenue (244) de manière à ce qu'elle puisse pivoter autour d'un deuxième axe (248), dans lequel le deuxième axe (248) est orienté sensiblement perpendiculairement au premier axe (246) et à la surface de retenue (244), **caractérisé**
**en ce que** la surface de retenue (244) est divisée par au moins un des éléments de retenue (240) en deux zones (254, 270), dans lequel la première zone (254) de la surface de retenue (244) est maintenue sur le corps support (242) et dans lequel la deuxième zone (270) est maintenue sur la première zone (254) de manière à pouvoir pivoter autour d'un troisième axe (268), dans lequel le troisième axe (268) est orienté sensiblement parallèlement à la surface de retenue (244) et sensiblement perpendiculairement au premier axe (246), et en ce que la première zone (254) présente un évidement (260), lequel s'étend de la zone du troisième axe (268) en direction du corps support (242), et
**en ce que** la deuxième zone (270) comporte une partie saillante (272) sensiblement complémentaire de l'évidement (260) de la première zone, dans lequel une butée (282) contre laquelle la deuxième zone (270) peut être amenée en appui est formée de préférence sur la première zone (254), ou
**en ce que** la première zone (314) présente une partie saillante (320), laquelle comporte à son extrémité libre un support pour le troisième axe (326), et
**en ce que** la deuxième zone (330) présente un évidement sensiblement complémentaire de la partie saillante (320) de la première zone (384), dans lequel une butée contre laquelle la deuxième zone (330) peut être amenée en appui est formée de préférence sur la première zone (314).

2. Système de rétraction chirurgical (10) selon la revendication 1, **caractérisé en ce que** la surface de retenue est divisée par au moins un des éléments de retenue (80) en deux zones (90, 100), dans lequel la première zone (90) de la surface de retenue est maintenue sur le corps support (84), dans lequel la deuxième zone (100) est montée mobile dans un plan parallèlement au premier axe par rapport à la première zone (90), dans lequel en particulier la deuxième zone (100) peut être positionnée à différentes intervalles par rapport à la partie support de l'élément de retenue le long du premier axe.

3. Système de rétraction chirurgical selon l'une quelconque des revendications 1 ou 2, **caractérisé en ce que** la surface de retenue (244) d'au moins un des éléments de retenue (240) s'amincit en forme de trapèze en direction de la première partie.

4. Système de rétraction chirurgical selon l'une quelconque des revendications 1 à 3, **caractérisé en ce que** la surface de retenue (68) d'au moins un des éléments de retenue (60) présente à son extrémité libre éloignée du bras écarteur (14, 16) une extension plus petite perpendiculairement au premier axe que dans une zone éloignée de celle-ci,
dans lequel, éventuellement, l'extrémité libre de la surface de retenue, laquelle extrémité est éloignée du bras écarteur (14, 16), s'amincit en direction d'une partie saillante (68) du type nervure.

5. Système de rétraction chirurgical selon la revendication 4, **caractérisé en ce que** l'extrémité libre de la surface de retenue, laquelle extrémité est éloignée du bras écarteur (14, 16), comporte une partie coudée (72).

6. Système de rétraction chirurgical selon l'une quelconque des revendications 1 à 5, **caractérisé en ce qu'**au moins deux éléments de retenue (240) sont agencés sur chaque bras écarteur (14, 16), dans lequel, éventuellement, les éléments de retenue (240) des deux bras écarteurs (14, 16) sont décalés l'un de l'autre, vus dans la direction longitudinale des bras écarteurs (14, 16).

7. Système de rétraction chirurgical selon l'une quelconque des revendications 1 à 6, **caractérisé en ce que** la distance entre le ou les éléments de retenue (240) et le dispositif de retenue (12) et de préférence également les uns par rapport aux autres le long du bras écarteur (14, 16) est réglable.

8. Système de rétraction chirurgical (200) selon l'une quelconque des revendications 1 à 7, **caractérisé en ce que** le système (200) comprend en outre un dispositif de levage (150), dans lequel le dispositif de levage (150) comporte un élément de maintien (152) qui s'étend sensiblement parallèlement au plan d'écartement du système et qui est retenu de préférence de manière détachable par une première extrémité à l'un des bras écarteurs, un dispositif d'appui (162, 168) s'étendant à partir de l'élément de maintien (152) et une plaque d'appui (164) maintenue à l'extrémité libre du dispositif d'appui (162, 168) et destinée à prendre appui sur la cage thoracique du patient, dans lequel le dispositif d'appui (162, 168) maintient la plaque d'appui (164) à une distance variable pouvant être prédéfinie du plan d'écartement.

9. Système de rétraction chirurgical (200) selon la revendication 8, **caractérisé en ce que** le dispositif d'appui (162, 168) comporte un dispositif de pivotement pourvu d'un bras pivotant (162) et un espaceur (168), dans lequel le bras pivotant (162) est monté à une première extrémité de manière articulée sur l'élément de maintien (152) du dispositif de levage (150), au voisinage du bras écarteur, et porte à sa deuxième extrémité opposée l'élément d'appui, et dans lequel l'espaceur (168) est retenu par une première extrémité, au voisinage de la deuxième extrémité de l'élément de maintien (152), sur celui-ci et s'appuie par sa deuxième extrémité sur le bras pivotant (162) et maintient ainsi la deuxième extrémité du bras pivotant (162), laquelle porte l'élément d'appui, à une distance pouvant être prédéfinie de manière variable par rapport à la deuxième extrémité de l'élément de maintien,
dans lequel, éventuellement, l'espaceur (168) est variable en longueur,
ou en variante l'espaceur (168) est maintenu de manière articulée par une première extrémité, au voisinage de la deuxième extrémité de l'élément de maintien (152), sur celui-ci, dans lequel plusieurs positions d'encliquetage sont formées sur le bras pivotant (162), dans lesquelles la deuxième extrémité de l'espaceur (168) peut être positionnée de manière détachable,
ou en variante l'espaceur (168) est relié de manière articulée par une première extrémité, au voisinage de la deuxième extrémité du bras pivotant, dans lequel plusieurs positions d'encliquetage sont formées sur l'élément de maintien (152), dans lesquelles la deuxième extrémité de l'espaceur peut être positionnée de manière détachable.

10. Système de rétraction chirurgical selon la revendication 9, **caractérisé en ce que** les positions d'encliquetage sont réalisées sous la forme d'un segment de crémaillère (170).

11. Système de rétraction chirurgical selon la revendication 9 ou 10, **caractérisé en ce que** l'élément de maintien (152) présente à sa deuxième extrémité un élément de préhension (160).

12. Système de rétraction chirurgical selon l'une quelconque des revendications 9 à 11, **caractérisé en ce que** l'élément de maintien (152) présente au voisinage de sa deuxième extrémité une ouverture à trou oblong (176) dans laquelle l'espaceur (168) est maintenu pivotant par sa première extrémité, dans lequel, éventuellement, l'espaceur (168) présente à sa première extrémité une partie de préhension (174), laquelle traverse l'ouverture à trou oblong (176) de l'élément de maintien (152).
